# EUROPEAN PATENT SPECIFICATION

(11) **EP 1 598 050 B1**
(45) Date of publication and mention of the grant of the patent: **10.09.2008**
(21) Application number: 04012179.0
(22) Date of filing: 22.05.2004
(51) Int. Cl.: A61K 8/49, A61Q 5/06, A61Q 5/08

(54) **Composition for bleaching and coloring keratin fibres**
Zusammensetzung zur Aufhellung und Färbung der Keratinfasern
Composition pour éclaircir et teindre les cheveux

(43) Date of publication of application: 23.11.2005
(73) Proprietor: KPSS-Kao Professional Salon Services GmbH, 64297 Darmstadt (DE)
(72) Inventor: Nöcker, Bernd, Dr., 64372 Ober-Ramstadt (DE); Wood, Jonathan, Dr., 69469 Weinheim (DE); Blumenschein, Katrin, 64753 Brombachtal-Hembach (DE)

(56) References cited:
- WO-A-02/074270
- FR-A- 2 844 269
- US-A- 5 188 639

## Description

The present invention concerns a new composition for the intensive, yet gentle bleaching and/or brightening and coloring of keratin fibers, especially human hair in the same process with improved color brilliance effect, less damaging and excellently durability.

Bleaching of human hair has been known for many decades. The bleaching of human hair customarily consists of a process with the following steps: Homogenous mixing of a water-free preparation, preferably a powder, comprising at least one compound with a bleaching or brightening effect, in particular a solid peroxide salt, preferably ammonium, potassium and/or sodium persulfate or earth alkali peroxide, with an aqueous hydrogen peroxide solution, application of this composition onto the hair, and rinsing after bleaching is completed.

Bleaching and coloring keratin fibers is customarily two different processes and achieved first by application of a bleaching composition and after removing the bleaching composition, a coloring composition known in the state of the art is applied. This is a time consuming process and often results in higher hair damage, Making available compositions achieving bleaching and coloring in a single process is very much desirable.

Previously it has been known that cationic direct dyes can be added into the bleaching compositions to carry out bleaching and coloring in the same process. However, the colors so obtained lacked usually durability and as well color brilliance.

WO 02/74270 A1 discloses bleaching compositions comprising bleach stable anionic and cationic direct dyes. The cationic and anionic dyes disclosed differ from the once according to claim 1.

US 5, 188,639 discloses hair dye compositions with a mixture of basic and acid dyes. The cationic dyes disclose are xanthene and not imidazole-azo dyes as in the present invention.

FR 2 844269 A1 discloses hyir dye compositions comprising peroxide and basic dyes same as the present application. The use of acidic dyes are not disclosed.

It has now been found, and it is the object of the present invention, that powder directs dyes of anionic and cationic character are added into compositions comprising compounds with bleaching and/or brightening effect to achieve bleaching and/or brightening and coloring in a single process.

Thus, according to the present invention bleaching and/or brightening and coloring compositions comprising at least one compound with bleaching and/or brightening effect, at least one cationic direct acting dyestuff and at least one anionic direct acting dyestuff selected from acid red 52, DC Violet 2, DC Red 27, DC Orange 4, DC Red 33 and DC Yellow 10.

It has further been found out that combination of the anionic and cationic dyestuffs has resulted in higher coloring performance than when they are used alone in coloring compositions. In other words synergistic effect is observed between anionic and cationic direct dyes.

The composition of the present invention has excellent gentle bleaching and/or brightening and coloring performance on hair. It improves as well the properties of the hair thus treated, in particular shine and elasticity of hair in addition to wet and dry combability, and volume of the hair.

Object of the invention is also a process for the bleaching and/or brightening and coloring of keratin fibers especially human hair with a preparation obtained by mixing a water-free composition (A), comprising at least a one compound with a bleaching and/or brightening effect and at least one anionic direct dye selected from acid red 52, DC Violet 2, DC Red 27, DC Orange 4, DC Red 33 and DC Yellow 10 and at least one cationic hair dye, with an aqueous hydrogen peroxide composition (B), which is applied onto hair and after 10 to 60 min of processing time at ambient temperature and/or at elevated temperature up to 45°C, rinsed off with water.

Peroxides are used as active components. Useful as such are in particular persulfates such as sodium and potassium persulfate, ammonium persulfate, earth alkali peroxides such as magnesium peroxide, melamine peroxide or urea peroxide or phtholimidoperoxy- hexanoic acid. The proportion of peroxides is at least 10 %, preferably at least 20 % to about 80 %, calculated to the total composition.

According to the invention, the bleaching compositions can also comprise 0.1 % to 10 % by weight, calculated to the total composition, of one or more ammonium salts.

Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium artrate, ammonium benzoate, ammonium acetate, ammonium formiate and ammonium lactate.

Preferred thereof are the ammonium phosphates, such as NH₄H₂PO₄, (NH₄)₂HPO₄, (NH₄)₂NaPO₄, NaNH₄HPO₄ or NH₄Na₂PO₄, ammonium chloride, ammonium sulfate and diammonium hydrogen citrate, as well as ammonium chloride, preferably in an amount from 0.1 % to 10 % by weight, calculated to the total composition.

As known from EP 609 796 A2, the ammonium compounds can also be used as sole bleaching agent in respectively higher amounts.

The proportion of the bleaching or brightening compounds in total preferably ranges from about 5 % to about 75 %, in particular about 25 % and about 60 % by weight, in reference to the brightening and bleaching powder.

According to the invention the compositions may further comprise the following direct acting anionic dyes :

Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 10, D&C Orange No. 11, D&C Red No. 21, D&C Yellow No. 7, D&C Yellow No. 8, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

The cationic dyestuffs with the following chemical structures are the ones according to the present invention. 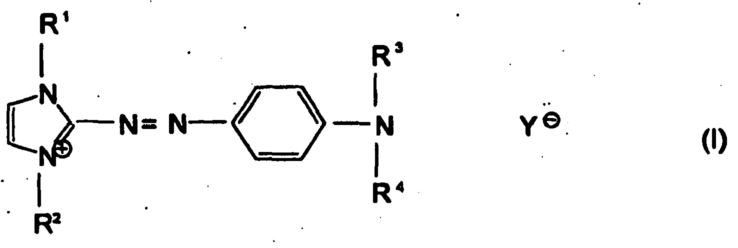 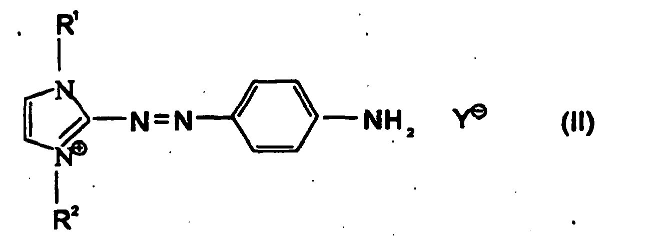 and 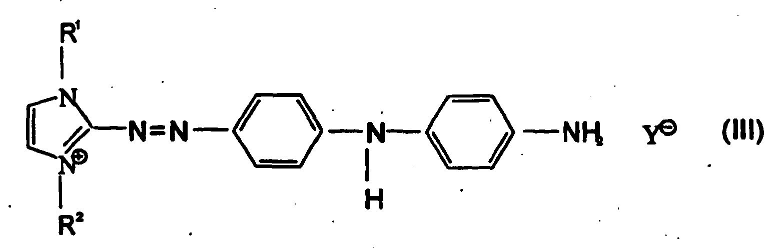 wherein R¹, R², R³ and R⁴ stand for hydrogen, a CH₃- or C₂H₅- group, and Y is an anion such as chloride, bromide, methosulfate.

The most preferred compounds are the ones according to the formula I, where R₁, R₂, R₃ and R₄ are methyl and Y is chloride and according to formula II where R₁ and R₂ are methyl and Y is chloride.

Suitable cationic dyestuffs are in principal those available on the market for cosmetic hair colouring applications. For this purpose, special reference is made to the PCT application WO 95/15144 of Ciba-Geigy AG.

According to the invention, bleaching and/ brightening and coloring composition comprises anionic dyes at a concentration of 0.1 to 7.5%, preferably 0.1 to 5%, more preferably 0.2 to 5% by weight calculated to total composition. Cationic dyestuffs are included into the compositions of the present invention at a concentration of 0.1 to 7.5%, preferably 0. to 5% and more preferably 0.2 to 5% by weight calculated to total composition.

Interestingly it has as well been found out that the ratio of acidic dyes to cationic dyes plays an important role for achieving especially resistance to washing and environmental effects. Accordingly, the ratio of cationic dyestuffs to acidic dyestuffs by weight is in the range of 2:1 to 1:10, preferably 1:1 to 1: 10 and more preferably 1: 1 to 1:7 and further more preferably 1:1 to 1:5.

Additionally other cationic dyestuffs can as well be used in addition to the cationic dyestuffs mentioned above. Some examples to those are:

Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

Additionally, the bleaching and/or brightening and coloring compositions of the present invention may comprise neutral dyes (HC dyes), so called nitro dyes for shading purposes. Concentration of those can typically be in the range from 0.01 to 2.5%, preferably 0.1 to 2% by weight calculated to total composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

In addition to the active component, the bleaching and/or brightening and coloring compositions also contain the components customarily used in such compositions.

In the event the preparation is a powder, in particular inert pulverulent carrier materials, these are for example, pyrogenic silicon dioxide, starch powder, etc., alkalizing agents, such as sodium metasilicate, surface-active substances, binding agents, etc.. In order to avoid repetition, reference is made to the respective standard literature, for example, K. Schrader, "Grundlagen und Rezepturen der Kosmetika", 2nd Ed. (1989, Hüthig Buchverlag), pages 815 to 823.

According to a preferred embodiment of the invention, the bleaching and/or brightening and coloring powders are used as dust-free products present as granules or agglomerates.

These can, for example, be those products disclosed in EP-A 560 088, which contain an oil or a liquid wax, whereby application of this oil or wax is preferably carried out in a spraying process.

According to the invention, it is also possible to use dust-free powders prepared according to EP-A 630 643 by agglomerating the initially pulverulent components by spraying onto these molten waxes or C₁₀-C₁₈-fatty acid alkanolamides, or by melting the same together at increased temperatures.

The particle sizes of the compositions according to the invention generally range below 1 mm, preferably below 500 micrometre, for example, less than 400 micrometre, in particular about 25 to about 100 µm, thus ensuring excellent processing capability, i.e. mixability with an aqueous hydrogen peroxide solution prior to application onto human hair.

The compositions of the present invention can comprise 0.001 % to 10 % by weight, calculated to the total composition, of at least one ubiquinone of the formula (I) 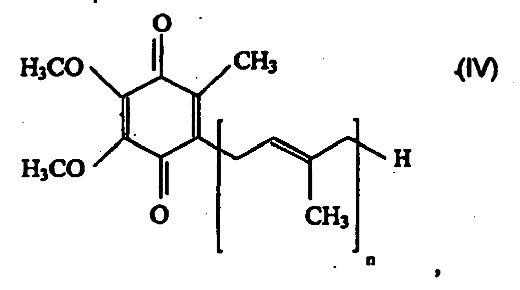 wherein n is a number from 1 to 10, and/or
a sphingolipid of the following formula II at a concentration of 0.001 % to 10 % by weight, calculated to the total composition, 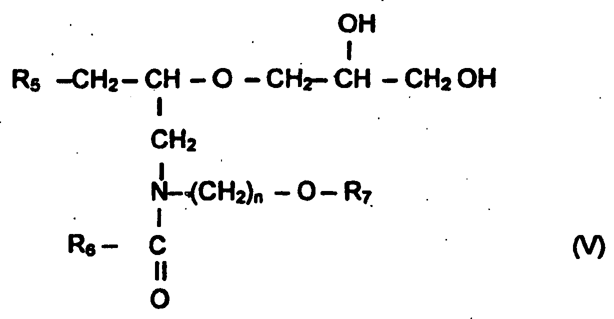 where R⁵ and R⁶ are independent from each other alkyl- or. alkenyl group mit 10 to 22 carbon atoms, R⁷ is methyl, ethyl, hydroxyethyl, n-propyl or isopropyl group and n is a number between 1 to 6, preferably 2 or 3. In number of previous applications the beneficial effects of those two ingredients in bleaching human hair are shown.

Preferred ubiquinone are those with n = 6 to 10, in particular ubiquinone 50, wherein n stands for 10, also known by the name "Coenzyme Q 10".

The preferred sphingolipid represented by the formula II is cetyl-PG hydroxyethyl palmitamide

The preferred use concentrations of the coenzymes Q 1 to Q 10 ranges from about 0.005 % to 5 %, in particular about 0.01% to 2.5 %, especially preferred about 0.05 % to 1 % by weight, calculated to the total bleaching and/or brightening and coloring powder composition (excluding the oxidation agent).

The preferred use concentrations of the spingolipids of the formula II ranges from about 0.005 % to 5 %, in particular about 0.01% to 2.5 %, especially preferred about 0.01 % to 1 % by weight, calculated to the total bleaching and/or brightening and coloring powder composition (excluding the oxidation agent).

Composition of the present invention can also contain cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quatemium-72, Quaternium-78, Quatemium-80, Quaternium-81, Quaternium-81, Quaternium-82, Quaternium-83 and Quaternium-84.

Typical concentration range for cationic polymers mentioned above can be 0.01 - 5% by weight, preferably 0.03 - 2.5% by weight and more preferably 0.05 - 1.5% by weight calculated to the total composition excluding aqueous peroxide lotion.

Nonionic polymers either natural or synthetic such as cellulose derivatives, hydroxyethylcellulose, xanthan gum, cellulose gums, poyvinylpyrrolidone can as well be part of the compositions.

Application of the composition is carried out in the customary manner known per se. The pulverulent brightening composition is mixed homogenously with a 3 percent to 12 percent hydrogen peroxide solution, preferably in a proportion of about one part by weight of the powder to about 0.5 to about 4 parts, in particular about 1 part to about 2.5 parts by weight of the peroxide solution or lotion, and subsequently left on the hair to process for about 10 to about 60 minutes, in particular about 20 to 30 minutes, depending on the temperature.

Application may also be carried out in a manner wherein the bleaching powder and the hydrogen peroxide solution are mixed with a cream base, this homogenous mixture then being applied onto the hair.

The composition is preferably adjusted at such levels that upon admixture with the aqueous hydrogen peroxide solution, it achieves a pH-value of about 8 to about 11.5, in particular between 9 and 11 in the ready-to-use mixture.

The following Examples illustrate the invention, but not limit.

### Example 1

### Bleaching coloring composition

| | |
|---|---|
| Hydroxyethylcellulose | 1.40% by weight |
| Cellulose gum | 3.10 |
| Xanthan gum | 0.30 |
| Tetrasodium EDTA | 3.00 |
| Sodium carbonate | 0.90 |
| Ammonium persulfate | 17.00 |
| Potassium persulfate | 35.00 |
| Sodium metasilicate | 8.00 |
| Corn starch | 7.70 |
| Diatomaceous Earth | 13.00 |
| Polyquaternium - 10 | 0.10 |
| Mineral oil | 9.20 |
| Fragrance | q.s. |
| DC Red 27 | 0.50 |
| Compound according to formula I R1=R2=R3=R4=methyl, Y=chloride | 0.50 |

The composition is prepared in the same way as disclosed in EP 560 088 A1.

The composition so obtained is mixed with hydrogen peroxide lotion (6% by weight, see example 3 for exact composition) at a ratio of 1:1.4 by weight (bleaching powder : hydrogen peroxide lotion)and mixed homogeneously and applied onto hair and left for 30 min at room temperature and subsequently rinsed of with water and shampooed with a conventional shampoo. In addition to this, the same compositions were prepared containing only DC Red 27 (1 %by weight) and containing only compound according to formula I R1=R2=R3=R4=methyl, Y=chloride (1% by weight) for comparative purposes. With those compositions bleaching and coloring is as well carried out in the same way as for the inventive composition as given above. The results are summarized in the table I.

**Table I: ΔE values after bleaching and coloring of hair**

| | ΔE |
|---|---|
| Inventive composition | 55 |
| | |
| Comparative composition (only with DC Red 27) | 51 |
| | |
| Comparative composition (only with compound of formula I R1=R2=R3=R4=methyl, Y=chloride) | 47 |

### Example 2

### Bleaching coloring composition

| | |
|---|---|
| Hydroxyethylcellulose | 1.00% by weight |
| Cellulose gum | 3.00 |
| Xanthan gum | 0.80 |
| Tetrasodium EDTA | 3.00 |
| Sodium carbonate | 0.90 |
| Ammonium persulfate | 20.00 |
| Potassium persulfate | 32.00 |
| Sodium metasilicate | 8.00 |
| Corn starch | 7.00 |
| Diatomaceous Earth | 14.00 |
| Polyquaternium - 10 | 0.30 |
| Mineral oil | 9.20 |
| Fragrance | q.s. |
| DC Orange 4 | 0.50 |
| Compound according to formula II R1=R2 =methyl, Y=chloride | 0.50 |

The composition is prepared in the same way as disclosed in EP 560 088 A1.

The composition so obtained is mixed with hydrogen peroxide lotion (6% by weight, see example 3 for exact composition) at a ratio of 1:1.4 by weight (bleaching podwer : hydrogen peroxide lotion) and mixed homogeneously and applied onto hair and left for 30 min at room temperature and subsequently rinsed of with water and shampooed with a conventional shampoo. In addition to this, the same compositions were prepared containing only DC Orange 4 (1 %by weight) and containing only compound according to formula II R1=R2 =methyl, Y=chloride (1% by weight) for comparative purposes. With those compositions bleaching and coloring is as well carried out in the same way as for the inventive composition as given above. The results are summarized in the table II.

**Table II: ΔE values after bleaching and coloring of hair**

| | ΔE |
|---|---|
| Inventive composition | 59 |
| | |
| Comparative composition (only with DC Orange 4) | 51 |
| | |
| Comparative composition (only with compound of formula II R1=R2= methyl, Y=chloride) | 54 |

From the above two examples and the results of the bleaching and coloring tests it is clear that the highest ΔE value is observed (being most effective) with the inventive compositions. All the comparative tests are showed less efficient performance. In addition synergistic effect is as well shown with the tests askeeping the total dyestuff concentration constant, the results observed with individual dyestuffs is always lower than when they are combined. This is a clear synergistic effect between anionic and cationic dyestuffs.

### Example 3

### Bleaching coloring composition

| | |
|---|---|
| Silicon dioxide | 16.17 (% by wt.) |
| Ammonium chloride | 14.70 |
| Sodium carbonate | 10.00 |
| Sodium metasilicate | 2.30 |
| Phthalimidoperoxyhexanic acid | 41.50 |
| Sodium persulfate | 15.20 |
| Coenzyme Q 10 | 0.10 |
| Cetyl PG hydroxyethyl palmitamide | 0.10 |
| DC Red 27 | 1.50 |
| Compound according to formula I R1=R2=R3=R4=methyl, Y=chloride | 0.50 |

This composition is mixed in a weight proportion of 1:2.5:2.5 with an H₂O₂ lotion b) and a cream base c):

### b) Hydrogen Peroxide Lotion

| | |
|---|---|
| Hydrogen peroxide | 6.00 (% by wt.) |
| Cetyl stearyl alcohol | 1.70 |
| Phosphoric acid | 0.50 |
| Sodium lauryl sulfate | 0.20 |
| Coenzyme Q 6 | 0.05 |
| Cetyl PG hydroxyethyl palmitamide | 0.10 |
| Disodium hydrogen phosphate | 0.10 |
| Salicylic acid | 0.10 |
| Water | ad 100.00 |

### c) Cream Base

| | |
|---|---|
| Cetyl stearyl alcohol | 11.0 (% by wt.) |
| Oleth-5 | 5.0 |
| Oleic acid | 2.5 |
| Stearamide MEA | 2.3 |
| Cocoamide MEA | 2.3 |
| Sodium cetyl stearyl sulfate | 1.2 |
| 1.2-Propyleneglycol | 1.0 |
| 1.2-Propyleneglycol stearate | 0.6 |
| Sodium lauryl sulfate | 0.5 |
| Wheat protein hydrolyzate | 0.9 |
| Organopolysiloxane | 0.4 |
| Cetyl PG hydroxyethyl palmitamide | 0.1 |
| Panthenol | 0.6 |
| Perfume | 0.4 |
| Complexing agent | 0.2 |
| Water | ad 100.0 |

The pH-value of this mixture was 9.0.

Upon application this mixture showed an excellent, even coloring effect with good wet and dry combability and excellent gloss, and improved elasticity; substantial hair damage was not apparent on strands of undamaged human hair.

### Example 4

### Bleaching and colouring powder

| | |
|---|---|
| Kieselguhr | 5.70(% by wt.) |
| Sodium carboxymethyl cellulose | 2.50 |
| Hydroxyethyl cellulose | 1.60 |
| Sodium lauryl sulfate | 2.50 |
| Sodium stearate | 1.60 |
| Protein hydrolyzate | 0.60 |
| Starch | 1.00 |
| Sodium carbonate | 0.60 |
| Sodium metasilicate | 9.00 |
| Polyvinyl pyrrolidone K90 | 3.20 |
| Coco fatty acid monoethanolamide | 9.80 |
| (NH₄)₂HPO₄ | 2.00 |
| Magnesium peroxide | 3.80 |
| Potassium persulfate | 8.00 |
| Sodium persulfate | 45.00 |
| Organopolysiloxane | 1.00 |
| Coenzyme Q 10 | 0.20 |
| Cetyl PG hydroxyethyl palmitamide | 0.10 |
| DC Orange 4 | 1.20 |
| Compound according to formula II R1=R2 =methyl, Y=chloride | 0.60 |

A dust-free powder is obtained, which shows good capabilities of being mixed with a known 6% H₂O₂-solution in a weight proportion of 1:1.4 (bleaching powder: hydrogen peroxide lotion). The pH-value of this mixture is 9.0.

Upon application, this mixture also showed an excellent, even colouring effect with good wet and dry combability and excellent gloss; substantial hair damage did not appear on strands of undamaged human hair. Elasticity of hair is enhanced as well dramatically.

### Example 5

### Dust-Free Bleaching Powder

| | |
|---|---|
| Silicon dioxide (diatomaceous earth) | 9.60 (% by wt.) |
| Silicon dioxide (pyrogenic SiO₂) | 5.00 |
| Sodium carboxymethyl cellulose | 3.50 |
| Urea | 2.00 |
| Sodium lauroyl sarcosinate | 0.80 |
| Sodium stearate | 1.20 |
| Sodium carbonate | 1.00 |
| Sodium metasilicate | 6.00 |
| Starch powder | 3.50 |
| Potassium persulfate | 48.00 |
| Magnesium peroxide | 4.00 |
| Organopolysiloxane | 1.00 |
| Coenzyme Q 10 | 0.10 |
| Cetyl PG hydroxyethyl palmitamide | 0.20 |
| Paraffin oil (Paraffinum perliquidum, | 11.50 |
| DAB 9) | |
| Acid red 52 | 1.00 |
| Compound according to formula I R1=R2=R3=R4=methyl, Y=chloride | 1.00 |

The powder was prepared by spraying the paraffin oil onto the basic powder mass in a fluidizing bed generator at about 20°C.
A dust-free powder is obtained, which shows good capability of being mixed with a known 6% H₂O₂ solution in a weight proportion of 1:1.
99% of the particles had a diameter of < 400 microns.

The coloring and bleaching effect, gloss and elasticity of the hair are excellent with good wet and dry combability, there was no evidence of essential hair damage.

### Example 6

### Dust-Free Bleaching Agglomerate

| | |
|---|---|
| Silicon dioxide (diatomaceous earth) | 3.00 (% by wt.) |
| Silicon dioxide (pyrogenic SiO₂) | 5.30 |
| Sodium carboxymethyl cellulose | 3.50 |
| Urea | 2.00 |
| Sodium lauroyl sarcosinate | 0.75 |
| Sodium stearate | 1.20 |
| Sodium carbonate | 1.00 |
| Sodium metasilicate | 6.00 |
| Starch powder | 4.40 |
| Sodium persulfate | 38.50 |
| Potassium persulfate | 13.00 |
| Magnesium peroxide | 4.00 |
| Cocomonoethanolamide | 15.00 |
| Cetyl PG hydroxyethyl palmitamide | 0.10 |
| Coenzyme Q 10 | 0.05 |
| Coenzyme Q 6 | 0.05 |
| Acid red 52 | 1.50 |
| Compound according to formula II R1=R2 =methyl, Y=chloride | 0.75 |

The powder was prepared by heating the above mixture to a temperature of 70°C to 75°C in a fluidizing bed generator and subsequent cooling.
A dust-free powder was obtained, which had good capability of being mixed with a known 9% H₂O₂ solution in a weight proportion of 1:1.5.
99% of the particles had a diameter of < 400 micrometre.
The bleaching and coloring effect achieved on the hair was even and excellent with good wet and dry combability of the hair; substantial hair damage was not in evidence.

## Claims

1. Non-aqueous bleaching and/or brightening and coloring composition for keratin fibers especially hair **characterized in that** it comprises at least one compound with a bleaching or brightening effect and at least one anionic direct dye selected from acid red 52, DC Violet 2, DC Red 27, PC Orange 4, DC Red 33 and DO Yellow 10 and at least one cationic direct dye selected from 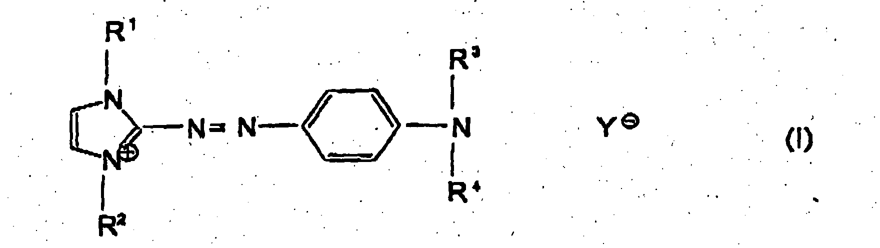 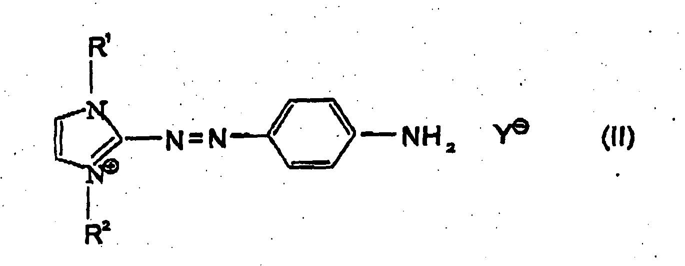 and 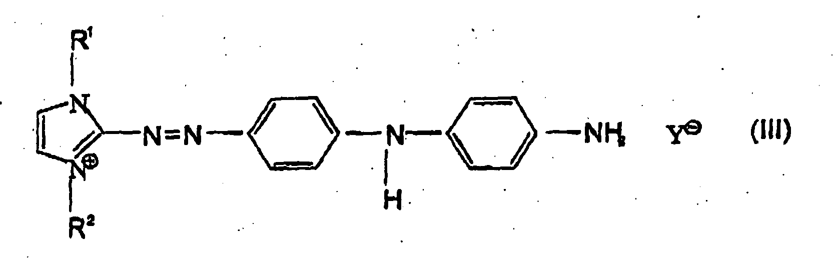 wherein R¹, R², R³ and R⁴ stand for hydrogen, a CH₃- or C₂H₅- group, and Y is an anion such as chloride, bromide, methosulfate.

2. Composition according to claim 1 **characterized in that** cationic dyestuff is 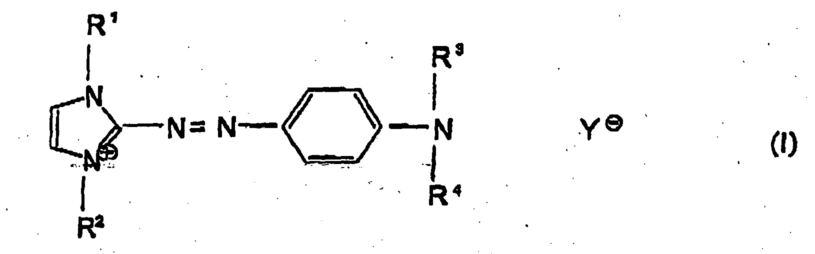 wherein R¹, R², R³ and R⁴ are CH₃- group, and Y is chloride.

3. Composition according to claim 1 **characterized in that** cationic dyestuff is 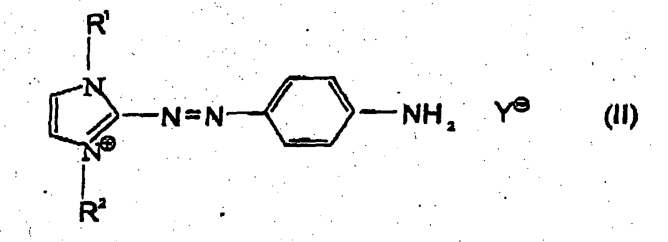 wherein R¹ and R² re CH₃- group, and Y is chloride.

4. Composition according to any of the preceding claims **characterized in that** it comprises at least one peroxide as a compound with a bleaching and/or brightening effect.

5. Composition according to any of the preceding claims **characterized in that** it comprises at least one ammonium salt as compound with a bleaching and/or brightening effect.

6. Composition according to any of the preceding claims **characterized in that** it comprises anionic dyestuffs at a concentration range from 0.1 to 7.5%. by weight calculated to total composition excluding hydrogen peroxide lotion.

7. Composition according to any of the preceding claims **characterized in that** it comprises cationic dyestuffs at a concentration range from 0.1 to 7.5% by weight calculated to total composition excluding hydrogen peroxide lotion.

8. Composition according to any of the preceding claims **characterized in that** it comprises at least one cationic dyestuff and at least one anionic dyestuff at a weight ratio of 2;1 to 1:10; preferably 1:1 to 1:7.

9. Composition according to any of the preceeding claims it comprises of at least one ubiquinone of the formula (I) 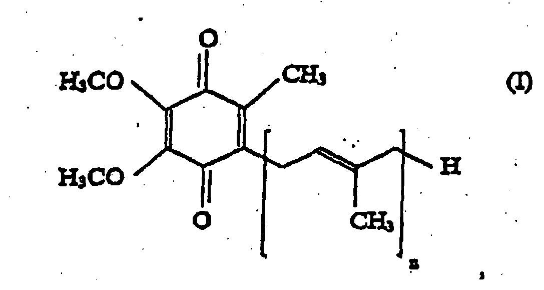 wherein n is a number from 1 to 10 and/or
at least one sphingolipid of the formula (II) 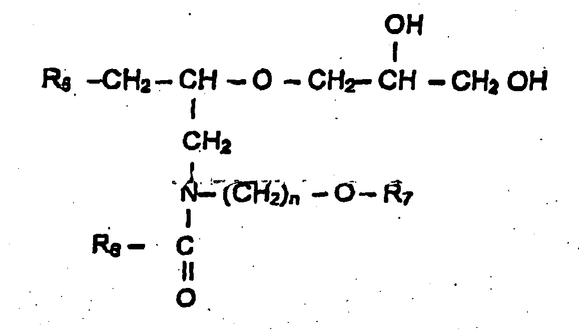 where R⁶ and R⁶ are independent from each other alkyl- or, alkenyl group mit 10 to 22 carbon atoms, R⁷ is methyl, ethyl, hydroxyethyl, n-propyl or isopropyl group and n is a number between 1 to 6, preferably 2 or 3.

10. Composition according to any of the preceding claims **characterized in that** it comprises at least one cationic polymer.

11. Process for the bleaching and/or brightening and coloring of keratin fibers, especially hair wherein a water-free composition (A), comprising at least one bleaching and/or brightening compound, and at least one anionic direct dye selected from acid Red 52, DC Violet 2, DCRed 27, DCorange 4, DCRed 33 and DC Yellow 10 and at least one cationic hair dye, is mixed with an aqueous hydrogen peroxide composition (B) and applied onto hair and after 10 to 60 min of processing time at ambient temperature and/or at elevated temperature up to 45°C, rinsed off from hair with water.

12. Use of composition according to claims 1 to 10 for bleaching and/or brightening and coloring of keratin fibers, especially hair.

## Patentansprüche

1. Nicht- wässrige Bleich- und/oder Aufhellungs- und Färbemittel-Zusammensetzung für Keratin-Fasern besonders Haare, **dadurch gekennzeichnet, dass** sie mindestens eine Verbindung mit einem Bleich-oder Aufhellungseffekt und mindestens einen anionischen direkt ziehenden Farbstoff, ausgewählt aus Acid Red 52, DC Violet 2, DC Red 27, DC Orange 4, DC Red 33 und DC Yellow 10, und mindestens einem kationischen direkt ziehenden Farbstoff, ausgewählt aus 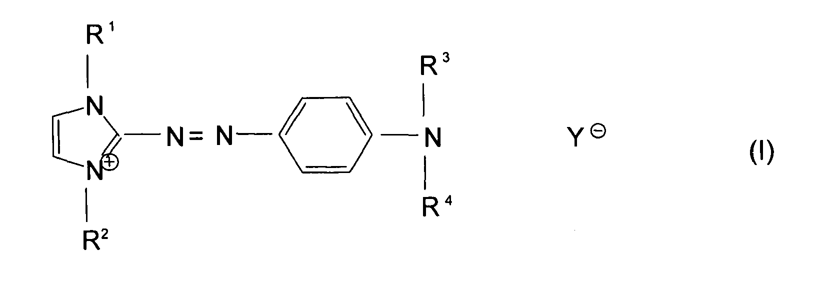 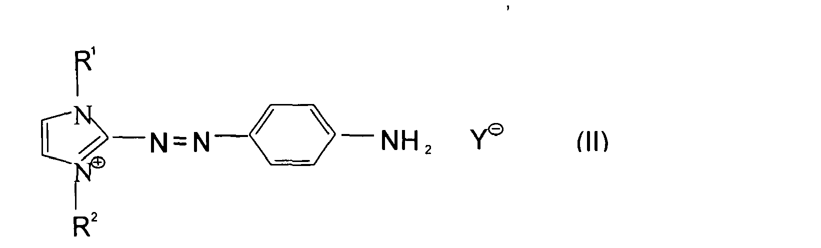 und 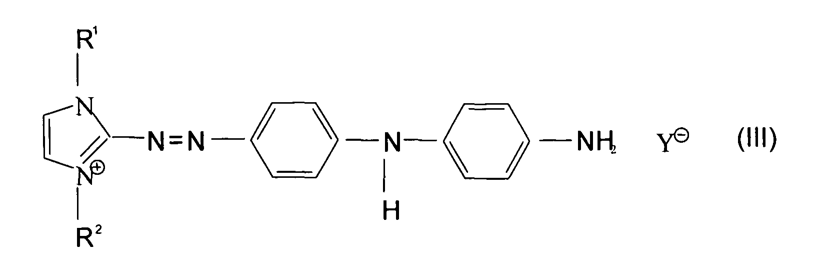 worin R¹, R², R³ und R⁴ für Wasserstoff, eine CH₃- oder C₂H₅- Gruppe stehen, und Y ein Anion wie Chlorid, Bromid und Methosulfat ist, enthält.

2. Zusammensetzung nach Anspruch 1, **dadurch gekennzeichnet, dass** der kationische Farbstoff 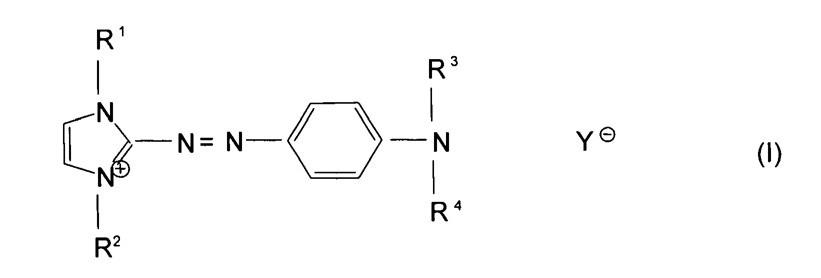 ist,
worin R¹, R², R³ und R⁴ CH₃-Gruppen sind, und Y Chlorid ist.

3. Zusammensetzung nach Anspruch 1, **dadurch gekennzeichnet, dass** der kationische Farbstoff 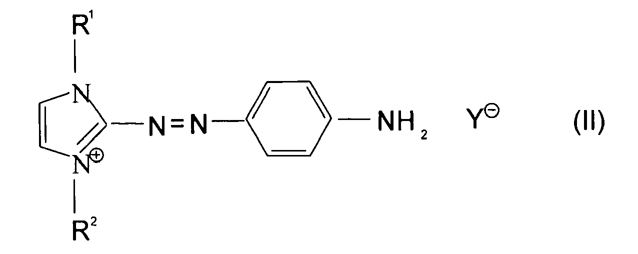 ist,
worin R¹ und R² CH₃- Gruppen sind, und Y Chlorid ist.

4. Zusammensetzung nach einem der vorhergehenden Ansprüche, **dadurch gekennzeichnet, dass** sie mindestens ein Peroxyd als eine Verbindung mit einem Bleich- und/oder Aufhellungseffekt enthält.

5. Zusammensetzung nach einem der vorhergehenden Ansprüche, **dadurch gekennzeichnet, dass** sie mindestens ein Ammoniumsalz als Verbindung mit einem Bleich und/oder Aufhellungseffekt enthält.

6. Zusammensetzung nach einem der vorhergehenden Ansprüche, **dadurch gekennzeichnet, dass** sie anionische Farbstoffe in einem Mengenbereich von 0,1 to 7,5% Gew.- %, berechnet auf die Gesamtzusammensetzung, ausgenommen Wasserstoffperoxyd-Lotion, enthält.

7. Zusammensetzung nach einem der vorhergehenden Ansprüche, **dadurch gekennzeichnet, dass** sie kationische Farbstoffe in einem Mengenbereich von 0,1 bis 7,5% Gew.- %, berechnet auf die Gesamtzusammensetzung, ausgenommen Wasserstoffperoxyd-Lotion, enthält.

8. Zusammensetzung nach einem der vorhergehenden Ansprüche, **dadurch gekennzeichnet, dass** sie mindestens einen kationischen Farbstoff und mindestens einen anionischen Farbstoff in einem Gewichtsverhältnis von 2:1 bis 1:10, vorzugsweise 1:1 bis 1:7, enthält.

9. Zusammensetzung nach einem der vorhergehenden Ansprüche, **dadurch gekennzeichnet, dass** sie mindestens ein Ubiquinone der Formel (I) 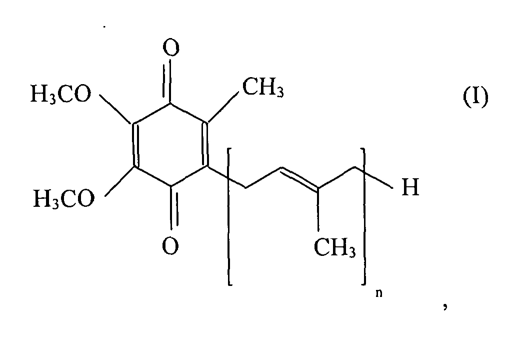 worin n eine Zahl von 1 bis 10 ist, und/oder
mindestens ein Sphingolipid der Formel (II) 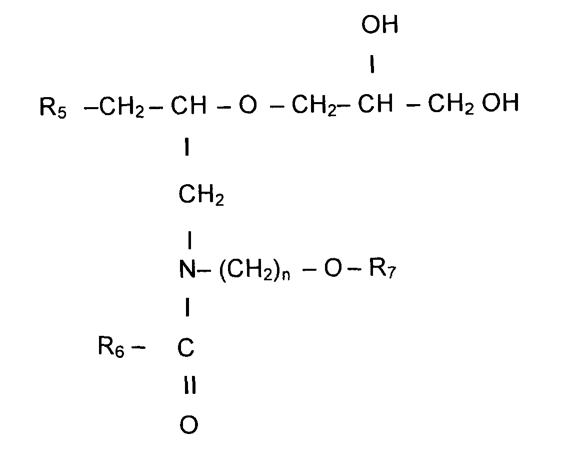 wobei R⁵ und R⁶ unabhängig voneinander Alkyl- oder. Alkenylgruppen mit 10 bis 22 Kohlenstoffatomen sind, R⁷ eine Methyl-, Ethyl-, Hydroxyethyl-, n-Propyl- oder Isopropylgruppe ist und n eine Zahl zwischen 1 bis 6, vorzugsweise 2 oder 3 ist, enthält.

10. Zusammensetzung nach einem der vorhergehenden Ansprüche, **dadurch gekennzeichnet, dass** sie mindestens ein kationisches Polymer enthält.

11. Verfahren zum Bleichen und/oder Aufhellen und Färben von Keratin-Fasern, insbesondere Haaren, wobei eine wasserfreie Zusammensetzung A, die mindestens eine bleichende und/ oder aufhellende Verbindung, und mindestens einen anionischen direkt ziehenden Farbstoff, ausgewählt aus Acid Red 52, DC Violet 2, DC Red 27, DC Orange 4, DC Red 33 und DC Yellow 10, und mindestens eine kationische Haarfarbe enthält, mit einer wässrigen Wasserstoffperoxyd- Zusammensetzung B gemischt wird, und auf das Haar aufgetragen wird, und nach 10 bis 60 Minuten Einwirkzeit bei normaler Temperatur und/oder erhöhter Temperatur bis zu 45°C, mit Wasser aus dem Haar ausgespült wird.

12. Verwendung der Zusammensetzung nach den Ansprüchen 1 bis 10 zum Bleichen und/oder Aufhellen und Färben von Keratin-Fasern, insbesondere Haaren.

## Revendications

1. Composition non aqueuse de blanchiment et/ou d'éclaircissement et de coloration pour des fibres de kératine en particulier des cheveux, **caractérisée en ce qu'**elle comprend au moins un composé ayant un effet de blanchiment ou d'éclaircissement et au moins un colorant anionique direct choisi parmi Acid Red 52, DC Violet 2, DC Red 27, DC Orange 4, DC Red 33 et DC Yellow 10, et au moins un colorant cationique direct choisi parmi 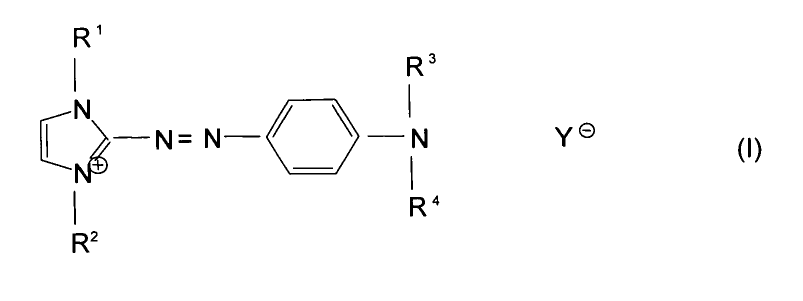 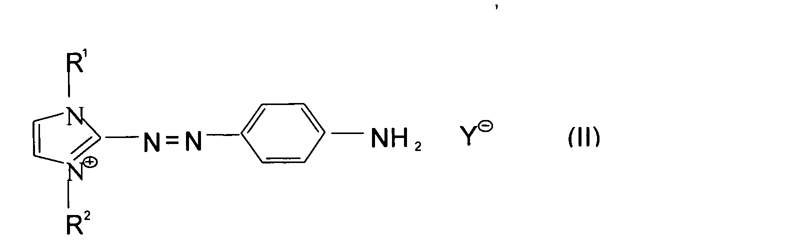 et 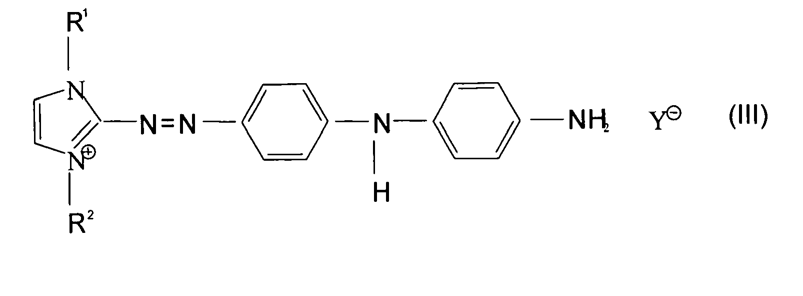 dans lesquels R¹, R², R³ et R⁴ représentent un hydrogène, un group CH₃- ou C₂H₅-, et Y est un anion tel qu'un chlorure, un bromure, un méthosulfate.

2. Composition selon la revendication 1, **caractérisée en ce que** la matière colorante cationique est 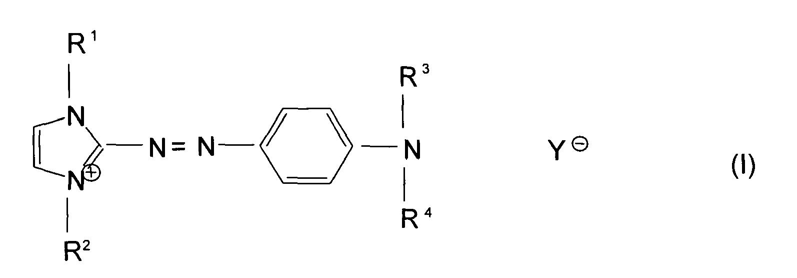 dans laquelle R¹, R², R³ et R⁴ sont un groupe CH₃-, et Y est un chlorure.

3. Composition selon la revendication 1, **caractérisée en ce que** la matière colorante cationique est 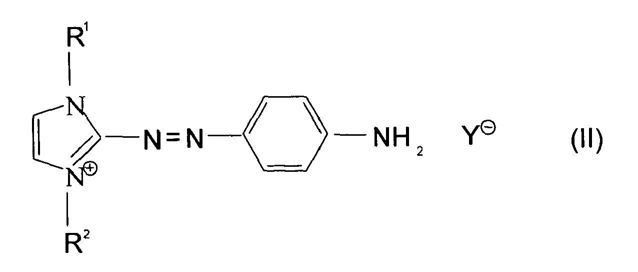 dans laquelle R¹ et R² sont un groupe CH₃-, et Y est un chlorure.

4. Composition selon l'une quelconque des revendications précédentes, **caractérisée en ce qu'**elle comprend au moins un peroxyde en tant que composé ayant un effet de blanchiment et/ou d'éclaircissement.

5. Composition selon l'une quelconque des revendications précédentes, **caractérisée en ce qu'**elle comprend au moins un sel d'ammonium en tant que composé ayant un effet de blanchiment et/ou d'éclaircissement.

6. Composition selon l'une quelconque des revendications précédentes, **caractérisée en ce qu'**elle comprend des matières colorantes anioniques à raison d'une concentration allant de 0,1 à 7,5% en poids calculée par rapport à la composition totale en excluant la lotion au peroxyde d'hydrogène.

7. Composition selon l'une quelconque des revendications précédentes, **caractérisée en ce qu'**elle comprend des matières colorantes cationiques à raison d'une concentration allant de 0,1 à 7,5% en poids calculée par rapport à la composition totale en excluant la lotion au peroxyde d'hydrogène.

8. Composition selon l'une quelconque des revendications précédentes, **caractérisée en ce qu'**elle comprend au moins une matière colorante cationique et au moins une matière colorante anionique à un rapport en poids de 2 :1 à 1 :10, de préférence de 1 :1 à 1 :7.

9. Composition selon l'une quelconque des revendications précédentes, **caractérisée en ce qu'**elle comprend au moins une ubiquinone de formule (I) 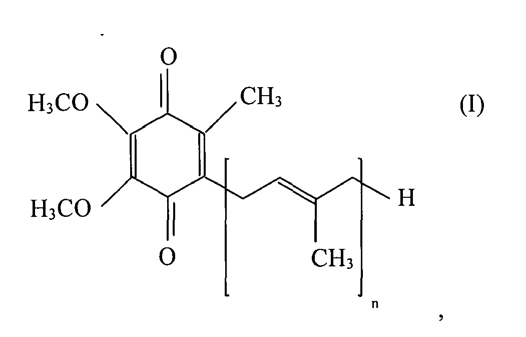 dans laquelle n est un nombre de 1 à 10 et/ou d'au moins un sphingolipide de formule (II) 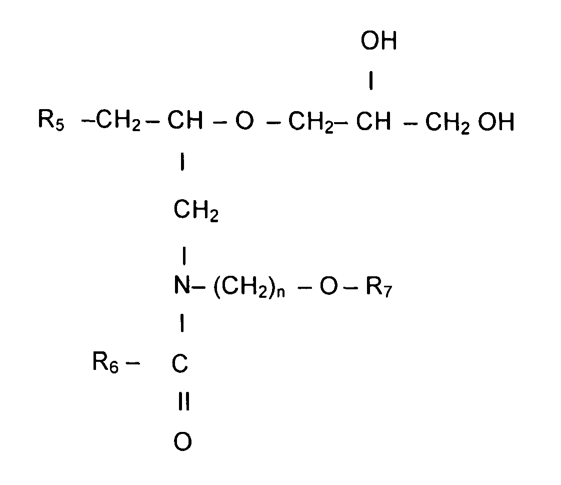 dans laquelle R⁵ et R⁶ sont, indépendamment l'un de l'autre, un groupe alkyle ou alcényle à 10 à 22 atomes de carbone, R⁷ est un groupe méthyle, éthyle, hydroxyéthyle, n-propyle ou isopropyle et n est un nombre compris entre 1 et 6, de préférence 2 ou 3.

10. Composition selon l'une quelconque des revendications précédentes, **caractérisée en ce qu'**elle comprend au moins un polymère cationique.

11. Procédé pour le blanchiment et/ou l'éclaircissement et la coloration de fibres de kératine, en particulier des cheveux, dans lequel une composition exempte d'eau (A), comprenant au moins un composé de blanchiment et/ou d'éclaircissement, et au moins un colorant anionique direct choisi parmi Acid Red 52, DC Violet 2, DC Red 27, DC Orange 4, DC Red 33 et DC Yellow 10, et au moins un colorant capillaire cationique, est mélangée à une composition aqueuse de peroxyde d'hydrogène (B) et appliquée sur les cheveux puis, après un temps de repos de 10 à 60 min à température ambiante et/ou à température élevée jusqu'à 40°C, éliminée des cheveux par rinçage à l'eau.

12. Utilisation d'une composition selon les revendications 1 à 10, pour le blanchiment et/ou l'éclaircissement et la coloration de fibres de kératine, en particulier des cheveux.
